# EUROPÄISCHE PATENTSCHRIFT

(11) **EP 0 562 435 B1**
(45) Veröffentlichungstag und Bekanntmachung des Hinweises auf die Patenterteilung: **07.06.1995**
(21) Anmeldenummer: 93104334.3
(22) Anmeldetag: 17.03.1993
(51) Int. Cl.: C07C 209/36, C07C 211/52, C07C 201/12, C07C 17/093, C07C 201/08

(54) **Verfahren zur Herstellung von 3,5-Difluoranilin**
Process for the preparation of 3,5-difluoroaniline
Procédé pour la préparation de la difluoro-3,5-aniline

(30) Priorität: 21.03.1992 DE 4209208
(43) Veröffentlichungstag der Anmeldung: 29.09.1993
(73) Patentinhaber: HOECHST AKTIENGESELLSCHAFT, 65926 Frankfurt am Main (DE)
(72) Erfinder: Schach, Thomas, Dr., D-64579Gernsheim (DE); Papenfuhs, Theodor, Dr., W-6000 Frankfurt am Main 90 (DE)

(56) Entgegenhaltungen:
- EP-A- 0 001 825
- EP-A- 0 460 639
- EP-A- 0 497 213
- US-A- 5 041 674
- CHEMICAL ABSTRACTS, vol. 52, no. 22, 25. November 1958, Columbus, Ohio, US; abstract no. 19987h, Seite 19987 ;Spalte 1 ;
- CHEMICAL ABSTRACTS, vol. 117, no. 23, 7. Dezember 1992, Columbus, Ohio, US; abstract no. 233580p, Seite 826 ;Spalte 2 ;
- TRANSACTIONS ILLINOIS ACADEMY OF SCIENCE Bd. 65, 1972, Seiten 75 - 80 D.R. DICKERSON ET AL. 'Reaction products of Pentachlorobenzene with potassium fluoride in dimethyl sulfoxide'
- JOURNAL OF THE AMERICAN CHEMICAL SOCIETY. Bd. 73, Nr. 1, 24. Januar 1951, GASTON, PA US Seiten 153 - 155 G.C. FINGER ET AL. 'Aromatic Fluorine Compounds. V. 1,3,5-Trifluorobenzene'

## Beschreibung

Die vorliegende Erfindung betrifft ein neues Verfahren zur Herstellung von 3,5-Difluoranilin, ausgehend von 2,4,5-Trichlornitrobenzol über das 5-Chlor-2,4-difluornitrobenzol, 1,3-Dichlor-4,6-difluorbenzol und 2,6-Dichlor-3,5-difluornitrobenzol.

3,5-Difluoranilin spielt eine wichtige Rolle als Synthesebaustein für pharmazeutische Produkte, wird aber auch im Bereich des Pflanzenschutzes als Zwischenprodukt benötigt.

Die Einführung zweier Fluorsubstituenten in 3- und 5-Position zu einer Amino- oder Nitrofunktion ist nur sehr schwer zu realisieren. Dafür kommen Synthesevarianten mit der technisch aufwendigen und dadurch sehr teuren Balz-Schiemann-Reaktion in Frage, neben einem weiteren Weg, der über Trinitrobenzol als Zwischenstufe führt. Neben ungünstigen Fluoridausbeuten ist dabei auch mit sehr großen Mengen an Nebenverbindungen zu rechnen, wobei zudem ein erheblicher sicherheitstechnischer Aufwand zu betreiben wäre (DE-OS 34 00 418). Eine weitere Synthesevariante führt über 2,4-Difluoranilin, das in einer vielstufigen Reaktionsfolge in nur mäßigen Ausbeuten in 3,5-Difluoranilin überführt werden kann (Finger G. C.; J. Am. Chem. Soc., 73, 153-155).

Es bestand daher ein Bedürfnis nach einem neuen Syntheseweg, der, basierend auf technisch einfach zu handhabenden Verfahrensstufen, ausgehend von einer leicht zugänglichen, im technischen Maßstab verfügbaren Ausgangsverbindung, die Herstellung des gewünschten 3,5-Difluoranilins in hohen Ausbeuten ermöglicht.

Es wurde nun überraschenderweise gefunden, daß man 3,5-Difluoranilin in vorteilhafter Weise und in guten Ausbeuten herstellen kann, indem man (1) 2,4,5-Trichlornitrobenzol mit einem Alkalimetallfluorid, wie Lithium-, Natrium-, Kalium-, Rubidium- oder Cäsiumfluorid, oder Mischungen daraus, in Gegenwart oder Abwesenheit eines polar-aprotischen Lösungsmittels, wie beispielsweise Dimethylformamid, Dimethylacetamid, Dimethylsulfoxid, Tetramethylensulfoxid, Dimethylsulfon, Diphenylsulfoxid, Diphenylsulfon, Sulfolan, N-Methylpyrrolidon oder 1,3-Dimethylimidazolin-2-on, in Abwesenheit oder Gegenwart eines Phasentransferkatalysators bei Temperaturen von etwa 100°C bis etwa 250°C, vorzugsweise von etwa 160°C bis etwa 200°C, umsetzt und nach Abfiltration ausgefallener Salze und fraktionierter Destillation der Rohlösung (2) das erhaltene 5-Chlor-2,4-difluornitrobenzol in Abwesenheit einer Lewissäure oder eines anderen Chlorierungskatalysators mit wasserfreiem Chlorgas bei Temperaturen von etwa 80°C bis etwa 250°C, vorzugsweise von etwa 100°C bis etwa 220°C, denitrierend chloriert zum 1,3-Dichlor-4,6-difluorbenzol, (3) diese Verbindung in Oleum mit Mischsäure (Schwefelsäure/Salpetersäure) bei Temperaturen von etwa 15°C bis etwa 80°C, vorzugsweise von etwa 20°C bis etwa 50°C nitriert zum 2,6-Dichlor-3,5-difluornitrobenzol und (4) diese Verbindung mit Wasserstoff oder einer Wasserstoff liefernden Verbindung in Gegenwart von Palladium als Katalysator und in Gegenwart einer organischen oder anorganischen Base zur Bindung des entstehenden Chlorwasserstoffs bei Temperaturen von etwa 40 bis etwa 250°C, vorzugsweise von etwa 70 bis etwa 150°C, reduziert.

Zu der letzten Stufe (4), betreffend die Überführung des 2,6-Dichlor-3,5-difluornitrobenzols in das 3,5-Difluoranilin, werden nachstehend im einzelnen noch folgende Verfahrensangaben gemacht:
Das als Katalysator zur Anwendung gelangende Palladium wird zweckmäßigerweise auf einem Trägermaterial eingesetzt. Geeignete Trägermaterialien sind beispielsweise Calciumcarbonat oder Bariumsulfat, vorzugsweise jedoch Aktivkohle.

Der Katalysator (Palladium) kann in Konzentrationen von etwa 0,5 bis etwa 30 Gewichtsprozent, bezogen auf das verwendete Trägermaterial, eingesetzt werden. Der Katalysator wird zweckmaßigerweise in Mengen von etwa 0,001 bis etwa 50 mmol Palladium pro Mol abzuspaltendem Chlor aus dem 2,6-Dichlor-3,5-difluornitrobenzol verwendet.

Als anorganische Basen zur Bindung des entstehenden Chlorwasserstoffs werden Ammoniak, Alkalimetall- oder Erdalkalimetallhydroxide, -oxide, -carbonate oder - hydrogencarbonate oder Mischungen daraus, wie beispielsweise die Natrium-, Kalium-, Calcium- oder Magnesiumverbindungen, verwendet.

Als organische Basen können Stickstoffbasen, wie beispielsweise Trialkyl(C₁-C₂₀)-amine, wobei die Alkylgruppen gleich oder verschieden sein können, beispielsweise Triethylamin, eingesetzt werden.

Die Basenkonzentration kann frei gewählt werden. Bevorzugt werden wäßrige Lösungen mit Konzentrationen von etwa 5 bis etwa 50 % der verwendeten Base. Dabei wird zweckmäßigerweise die Konzentration der Base so gewählt, daß das entstehende Ammonium-, Alkalimetall- oder Erdalkalimetallchlorid bei Aufarbeitungstemperatur noch vollständig in Lösung verbleibt.

Als Reduktionsmittel wird bevorzugt Wasserstoff eingesetzt. Es können aber auch andere Reduktionsmittel, die als Wasserstofflieferant dienen können, verwendet werden, wie beispielsweise Hydrazinhydrat, Glykole, wie Ethylenglykol, höherwertige Alkohole, wie Glycerin, einwertige aliphatische Alkohole, wie Methanol oder Ethanol, oder Formiate.

Die letztgenannte Umsetzung (Stufe 4) kann in An- oder Abwesenheit von Luftsauerstoff durchgeführt werden.

Wird Wasserstoff als Reduktionsmittel verwendet, arbeitet man unter einer Wasserstoffatmosphäre unter Druck. Bevorzugt sind hierbei Wasserstoffdrücke von etwa 0,1 bis etwa 50 bar.

Die Reduktion kann bei Temperaturen von etwa 40 bis etwa 250°C durchgeführt werden, wobei bevorzugt bei Temperaturen von etwa 70 bis etwa 150°C gearbeitet wird. Zu tiefe Temperaturen ergeben eine langsame und unvollständige Reaktion, während zu hoch gewählte Temperaturen Fluoridabspaltung zur Folge haben können.

Um die besagte Fluoridabspaltung, die besonders gegen Ende der Reaktion auftritt, möglichst gering zu halten, wird die Reduktion bevorzugt bei Umsätzen von 90 - 95 % abgebrochen und die so erhaltene Rohlösung fraktioniert destilliert. Nicht vollständig umgesetzte Zwischenprodukte können dabei in der Folgereaktion wieder eingesetzt werden.

Der bei der Reaktion anfallende gebrauchte Katalysator kann unbehandelt weiterverwendet oder durch bekannte Reinigungsverfahren, wie beispielsweise durch Wasserdampf, gereinigt werden.

Günstigere Katalysatorausnutzungen lassen sich durch eine sukzessive Reduktion erreichen, indem man zuerst bei relativ niedrigen Temperaturen innerhalb des angegebenen Temperaturbereichs und ohne Base die Nitrogruppe zur Aminogruppe reduziert und anschließend durch Zugabe einer Base bei höherer Temperatur innerhalb des angegebenen Temperaturbereichs die Chlorabspaltung einleitet.

Als Ausgangsverbindung der Reduktion können neben 3,5-Difluor-2,6-dichlornitrobenzol auch Mischungen verschiedener Dichlordifluornitrobenzole eingesetzt werden, insbesondere Mischungen, wie sie bei der Nitrierung von 1,3-Dichlor-4,6-difluorbenzol anfallen.

Die Reinigung der nach der Reduktion erhaltenen Rohlösung erfolgt bevorzugt durch Wasserdampfdestillation und/oder fraktionierte Destillation; gegebenenfalls kann eine Reinigung durch Kristallisation angeschlossen werden.

Die Ausbeute an 3,5-Difluoranilin ist, bezogen auf das unmittelbare Vorprodukt 2,6-Dichlor-3,5-difluornitrobenzol, sehr gut.

Das 2,6-Dichlor-3,5-difluornitrobenzol ist nach Stufe 3 in guten Ausbeuten (75 - 95 %) und Selektivitäten (87:12; 3,5-Difluor-2,6-dichlornitrobenzol:2,6-Difluor-3,5-dichlornitrobenzol) durch Nitrierung von 1,3-Dichlor-4,6-difluorbenzol zugänglich. (Siehe auch Trans. III. State Acad. Sci., 65, (1972) 75 - 80.)

Als Ausgangsverbindungen für die Nitrierung kann sowohl mit reinem 1,3-Dichlor-4,6-difluorbenzol als auch mit Mischungen aus 1,3-Dichlor-4,6-difluorbenzol und 1,3-Dichlor-2,4-difluorbenzol gearbeitet werden. Zur Nitrierung des 1,3-Dichlor-4,6-difluorbenzols werden bevorzugt Mischungen aus Oleum, H₂SO₄ und HNO₃, die so berechnet sind, daß am Ende der Reaktion eine 95 - 100 %ige Schwefelsäure entsteht, verwendet. Das bei der Nitrierung entstandene Produkt (2,6-Dichlor-3,5-difluornitrobenzol) kann nach dem Verdünnen der Schwefelsäure bei Temperaturen von etwa 30 bis etwa 45°C als flüssige Phase abgetrennt werden. Zweckmäßigerweise wird die organische Phase vor dem Abtrennen in einem zusätzlichen Lösungsmittel aufgenommen. Als Lösungsmittel kommen hierfür beispielsweise Toluol, Xylol, niedere Alkane, Ether und Polyether in Betracht. Die so erhaltene Rohlösung wird nach dem Neutralwaschen bevorzugt ohne weitere Vorbehandlung in der Folgestufe eingesetzt. Eine Trennung der entstandenen Isomere durch fraktionierte Destillation ist jedoch ebenfalls möglich.

Die für diese Nitrierung benötigte Ausgangsverbindung 1,3-Dichlor-4,6-difluorbenzol läßt sich nach Stufe 2 (Einwirkung von Chlor in Abwesenheit einer Lewissäure auf 5-Chlor-2,4-difluornitrobenzol) in sehr guten Ausbeuten herstellen. Die Einwirkung des Chlors kann hierbei auf 5-Chlor-2,4-difluornitrobenzol allein oder auf eine Mischung aus der letztgenannten Verbindung und 3-Chlor-2,4-difluornitrobenzol (Gewichtsverhältnis 85:14) erfolgen. Die (denitrierende) Chlorierung kann sowohl diskontinuierlich als auch kontinuierlich vorgenommen werden, wobei die kontinuierliche (denitrierende) Chlorierung bevorzugt wird (kontinuierliche Zugabe des 5-Chlor-2,4-difluornitrobenzols oder der genannten Mischung und kontinuierliche Abnahme des gebildeten 1,3-Dichlor-4,6-difluorbenzols).

Als Phasentransferkatalysatoren können in Stufe 1 (Umsetzung von 2,4,5-Trichlornitrobenzol mit einem Alkalimetallfluorid in einem polar-aprotischen Lösungsmittel) Tetraalkyl(C₁-C₁₈)-ammoniumchloride oder -bromide, Tetraalkyl (C₁-C₁₈)-phosphoniumchloride oder -bromide, Tetraphenylphosphoniumchlorid oder -bromid, [(Phenyl)ₘ(alkyl(C₁-C₁₈)ₙ]-phosphoniumchloride oder -bromide, wobei m = 1 bis 3, n = 3 bis 1 und m+n = 4 ist, verwendet werden.

Das in erster Stufe erhaltene 5-Chlor-2,4-difluornitrobenzol kann auch durch Chlorieren des in technischem Maßstab verfügbaren 2,4-Difluornitrobenzols in guten Ausbeuten hergestellt werden, indem man beispielsweise 4801 g (30.2 mol) 2,4-Difluornitrobenzol mit 82 g FeCl₃ vorlegt, die Reaktionslösung auf 80°C erwärmt, durch die Lösung Chlor leitet, nach 78 Stunden die Reaktion abbricht, die Reaktionslösung mit Natriumsulfitlösung ausschüttelt, mit Wasser neutral wäscht und fraktioniert destilliert.

Das in zweiter Stufe erhaltene 1,3-Dichlor-4,6-difluorbenzol kann auch durch Chlorierung von 1,3-Difluorbenzol bzw. von 1-Chlor-2,4-difluorbenzol in guten Selektivitäten und Ausbeuten hergestellt werden.

Die nachstehenden Beispiele dienen zur Erläuterung des erfindungsgemäßen Verfahrens ohne es darauf zu beschränken.

### Stufe 1

### Herstellung von 5-Chlor-2,4-difluornitrobenzol

In einem 2 l Dreihalskolben mit Innenthermometer, Blattrührer und Rückflußkühler werden 452,8 g (2 mol) 2,4,5-Trichlornitrobenzol, 232,4 g (4,0 mol) Kaliumfluorid, 60,8 g (0,4 mol) Cäsiumfluorid und 632 g Sulfolan vorgelegt. Die Reaktionssuspension wird auf 160°C erhitzt und für 8 Stunden bei dieser Temperatur kräftig gerührt. Anschließend wird auf Raumtemperatur abgekühlt, die ausgefallenen Salze abgenutscht und die Rohlösung fraktioniert destilliert.

Es werden 267,1 g 5-Chlor-2,4-difluornitrobenzol, was einer Ausbeute von 69 % der Theorie entspricht, erhalten.

### Stufe 2

### Herstellung von 1,3-Dichlor-4,6-difluorbenzol

3 kg (15,5 mol) eisenfreies, trockenes 5-Chlor-2,4-difluornitrobenzol werden in einem 2 l Vierhalskolben mit Rührer, Tropftrichter, Gaseinleitungsrohr und aufgesetzter Kolonne mit Rückflußkühler vorgelegt. Nach Erhitzen der Reaktionslösung auf 200°C wird Chlor mit einer Geschwindigkeit von 15 l/h durch die Lösung geleitet. Nach 4 - 6 Stunden beginnt die Lösung zu sieden und nach weiteren 2 - 4 Stunden kann am Kolonnenkopf das gebildete 1,3-Dichlor-4,6-difluorbenzol abgenommen werden. Entsprechend der abgenommenen Produktmenge wird gleichzeitig frisches 5-Chlor-2,4-difluornitrobenzol der Reaktion zugeführt.

Die Ausbeuten liegen über 90 % der Theorie an 1,3-Dichlor-4,6-difluorbenzol, bezogen auf umgesetztes 5-Chlor-2,4-difluornitrobenzol.

### Stufe 3

### Herstellung von 2,6-Dichlor-3,5-difluornitrobenzol

In einem 2 l Vierhalskolben mit Blattrührer, Rückflußkühler und 500 ml Tropftrichter werden 475,7 g (2,6 mol) 1,3-Dichlor-4,6-difluorbenzol in 981,8 g Oleum (20 % SO₃) suspendiert. Anschließend werden innerhalb von 4 Stunden 595,7 g Mischsäure (66 % H₂SO₄/34 % HNO₃) langsam zugetropft sodaß die Reaktionstemperatur nicht über 30°C steigt. Nach beendeter Zugabe wird die Reaktionslösung für weitere 2 Stunden bei 30 - 40°C nachgerührt, auf 1500 g Eis gegeben, auf 40°C gebracht und die organische Phase abgetrennt. Die organische Phase wird anschließend mit 100 g Toluol versetzt, mit Na₂CO₃-Lösung behandelt und mit Wasser neutralgewaschen. Die so erhaltene Rohlösung kann ohne weitere Aufarbeitung in die Folgestufe eingesetzt werden.

Erhalten werden
633,0 g Rohlösung, davon sind
547,0 g Rohprodukt (86 % davon 2,6-Dichlor-3,5-difluornitrobenzol)
(11 % davon 3,5-Dichlor-2,6-difluornitrobenzol)
Die Ausbeute an 2,6-Dichlor-3,5-difluornitrobenzol beträgt 79,4 % der Theorie.

### Stufe 4a

### Herstellung von 3,5-Difluoranilin

Es werden 547 g rohes 2,6-Dichlor-3,5-difluornitrobenzol (bestehend aus 86 % 2,6-Dichlor-3,5-difluornitrobenzol und 13 % 3,5-Dichlor-2,6-difluornitrobenzol) in 200 g Toluol und 25 g Pd/C (5 %ig, 50 % wasserfeucht) im Reaktionsgefäß (Autoklav) vorgelegt. Die Mischung der Reaktionskomponenten wird auf 45°C aufgeheizt. Bei dieser Temperatur wird dann mit Wasserstoff zum entsprechenden Amin reduziert. Ist keine Wasserstoffaufnahme mehr zu beobachten, wird eine Stunde bei gleicher Temperatur nachgerührt, auf Raumtemperatur abgekühlt und die Reaktionslösung mit 1064 g Natronlauge (20 %ig) versetzt. Nach dem Aufheizen auf 80°C erfolgt die reduktive Entchlorierung, bis keine Wasserstoffaufnahme mehr zu beobachten ist. Anschließend wird der Katalysator vom Reaktionsgemisch abgesaugt, die organische Phase abgetrennt und fraktioniert destilliert.

Erhalten werden
184,4 g 3,5-Difluoranilin (Ausbeute 69,2 % der Theorie)
und
19,8 g 2,6-Difluoranilin (Ausbeute 58,2 % der Theorie).

### Stufe 4b

### Herstellung von 3,5-Difluoranilin

Es werden 547 g rohes 2,6-Dichlor-3,5-difluornitrobenzol (bestehend aus 86 % 2,6-Dichlor-3,5-difluornitrobenzol und 13 % 3,5-Dichlor-2,6-difluornitrobenzol) in 100 g Toluol zusammen mit 17,5 g Pd/C (5 %ig, 50 % wasserfeucht) 104,8 g MgO in 450 g Wasser im Reaktionsgefäß (Autoklav) vorgelegt. Die Mischung der Reaktionskomponenten wird auf 45°C aufgeheizt und bei dieser Temperatur mit Wasserstoff zum entsprechenden Amin reduziert. Bei nachlassender Reaktion wird die Temperatur sukzessive auf 120 - 130°C erhöht, wodurch sich die Wasserstoffaufnahme wieder verstärkt und die vorhandenen Chloratome reduktiv abgespalten werden. Nach beendeter Reaktion wird eine Stunde bei gleicher Temperatur nachgerührt, auf Raumtemperatur abgekühlt, die Rohlösung mit Wasserdampf destilliert und die erhaltene organische Phase abgetrennt und fraktioniert destilliert.

Ausbeute:
29,1 g (80,1 %) 2,6-Difluoranilin
237,8 g (89,9 %) 3,5-Difluoranilin
Bezogen auf Stufe 4b beträgt die Ausbeute über die letzten drei Stufen 63,8 % der Theorie.

Bezogen auf Stufe 4b beträgt die Ausbeute über alle Stufen 44 % der Theorie.

## Patentansprüche

1. Verfahren zur Herstellung von 3,5-Difluoranilin, dadurch gekennzeichnet, daß man
(1) 2,4,5-Trichlornitrobenzol mit einem Alkalimetallfluorid in Gegenwart oder Abwesenheit eines polar-aprotischen Lösungsmittels bei Temperaturen von etwa 100°C bis etwa 250°C umsetzt und nach Abfiltration ausgefallener Salze und fraktionierter Destillation der Rohlösung
(2) das erhaltene 5-Chlor-2,4-difluornitrobenzol in Abwesenheit einer Lewissäure oder eines anderen Chlorierungskatalysators mit wasserfreiem Chlorgas bei Temperaturen von etwa 80 bis etwa 250°C denitrierend chloriert zum 1,3-Dichlor-4,6-difluorbenzol,
(3) diese Verbindung in Oleum mit Mischsäure (Schwefelsäure/Salpetersäure) bei Temperaturen von etwa 15 bis etwa 80°C nitriert zum 2,6-Dichlor-3,5-difluornitrobenzol und
(4) diese Verbindung mit Wasserstoff in Gegenwart von Palladium als Katalysator und in Gegenwart einer anorganischen oder organischen Base bei Temperaturen von etwa 40 bis etwa 250°C reduziert.

2. Verfahren nach Anspruch 1, dadurch gekennzeichnet, daß man in Stufe 1 mit Lithium-, Natrium-, Kalium-, Rubidium- oder Cäsiumfluorid oder Mischungen daraus umsetzt.

3. Verfahren nach mindestens einem der Ansprüche 1 und 2, dadurch gekennzeichnet, daß man in Stufe 1 bei Temperaturen von etwa 160 bis etwa 200°C umsetzt.

4. Verfahren nach mindestens einem der Ansprüche 1 bis 3, dadurch gekennzeichnet, daß man in Stufe 1 in Dimethylformamid, Dimethylacetamid, Dimethylsulfoxid, Tetramethylensulfoxid, Dimethylsulfon, Diphenylsulfoxid, Diphenylsulfon, Sulfolan, N-Methylpyrrolidon oder 1,3-Dimethylimidazolin-2-on als polar-aprotischem Lösungsmittel umsetzt.

5. Verfahren nach mindestens einem der Ansprüche 1 bis 4, dadurch gekennzeichnet, daß man in Stufe 1 in Gegenwart eines Tetraalkyl(C₁-C₁₈)-ammoniumchlorids oder -bromids, Tetraalkyl(C₁-C₁₈)-phosphoniumchlorids oder -bromids, Tetraphenylphosphoniumchlorids oder -bromids, [(Phenyl)ₘ(alkyl(C₁-C₁₈)ₙ]-phosphoniumchlorids oder -bromids, wobei m = 1 bis 3, n = 3 bis 1 und m + n = 4 ist, als Phasentransferkatalysator umsetzt.

6. Verfahren nach mindestens einem der Ansprüche 1 bis 5, dadurch gekennzeichnet, daß man in Stufe 2 bei Temperaturen von etwa 100 bis etwa 220°C denitrierend chloriert.

7. Verfahren nach mindestens einem der Ansprüche 1 bis 6, dadurch gekennzeichnet, daß man die denitrierende Chlorierung in Stufe 2 kontinuierlich durchführt.

8. Verfahren nach mindestens einem der Ansprüche 1 bis 7, dadurch gekennzeichnet, daß man in Stufe 3 mit einer Mischsäure aus 66 % H₂SO₄ und 34 % HNO₃ in Oleum nitriert.

9. Verfahren nach mindestens einem der Ansprüche 1 bis 8, dadurch gekennzeichnet, daß man in Stufe 3 bei Temperaturen von etwa 20 bis etwa 50°C nitriert.

10. Verfahren nach mindestens einem der Ansprüche 1 bis 9, dadurch gekennzeichnet, daß man in Stufe 4 Glykole, höherwertige Alkohole, einwertige aliphatische Alkohole oder Formiate als Reduktionsmittel einsetzt.

11. Verfahren nach mindestens einem der Ansprüche 1 bis 10, dadurch gekennzeichnet, daß man in Stufe 4 bei Verwendung von Wasserstoff als Reduktionsmittel unter einer Wasserstoffatmosphäre unter Druck arbeitet.

12. Verfahren nach mindestens einem der Ansprüche 1 bis 11, dadurch gekennzeichnet, daß man bei Verwendung von Wasserstoff als Reduktionsmittel unter einer Wasserstoffatmosphäre unter einem Druck von etwa 0,1 bis etwa 50 bar arbeitet.

13. Verfahren nach mindestens einem der Ansprüche 1 bis 12, dadurch gekennzeichnet, daß man in Stufe 4 das Palladium auf einem Trägermaterial einsetzt.

14. Verfahren nach mindestens einem der Ansprüche 1 bis 13, dadurch gekennzeichnet, daß man in Stufe 4 das Palladium auf Calciumcarbonat, Bariumsulfat oder Aktivkohle als Trägermaterial einsetzt.

15. Verfahren nach mindestens einem der Ansprüche 1 bis 14, dadurch gekennzeichnet, daß man in Stufe 4 das Palladium in Mengen von etwa 0,001 bis etwa 50 mmol pro Mol abzuspaltendem Chlor verwendet.

16. Verfahren nach mindestens einem der Ansprüche 1 bis 15, dadurch gekennzeichnet, daß man in Stufe 4 das Palladium in Konzentrationen von etwa 0,5 bis etwa 30 Gew.-%, bezogen auf das angewandte Trägermaterial, einsetzt.

17. Verfahren nach mindestens einem der Ansprüche 1 bis 16, dadurch gekennzeichnet, daß man in Stufe 4 in Gegenwart von Ammoniak oder eines Alkalimetall- oder Erdalkalimetallhydroxids, -oxids, -carbonats oder - hydrogencarbonats oder Mischungen daraus reduziert.

18. Verfahren nach mindestens einem der Ansprüche 1 bis 17, dadurch gekennzeichnet, daß man die Reduktion der Stufe 4 nach einem Umsatz von 90 bis 95 % abbricht und die so erhaltene Rohlösung fraktioniert destilliert.

## Claims

1. A process for preparing 3,5-difluoroaniline, wherein
(1) 2,4,5-trichloronitrobenzene is reacted with an alkali metal fluoride in the presence or absence of a polar aprotic solvent at temperatures of from about 100°C to about 250°C, and, after filtering off precipitated salts and fractional distillation of the crude solution,
(2) the resulting 5-chloro-2,4-difluoronitrobenzene is chlorinated with denitration, in the absence of a Lewis acid or of another chlorination catalyst, using anhydrous chlorine gas at temperatures of from about 80 to about 250°C, to give 1,3-dichloro-4,6-difluorobenzene,
(3) this compound is nitrated in oleum containing mixed acid (sulfuric acid/nitric acid), at temperatures of from about 15 to about 80°C, to give 2,6-dichloro-3,5-difluoronitrobenzene, and
(4) this compound is reduced with hydrogen in the presence of palladium as catalyst and in the presence of an inorganic or organic base at temperatures of from about 40 to about 250°C.

2. The process as claimed in claim 1, wherein the reaction in stage 1 is carried out using lithium fluoride, sodium fluoride, potassium fluoride, rubidium fluoride or cesium fluoride, or mixtures thereof.

3. The process as claimed in at least one of claims 1 and 2, wherein the reaction in stage 1 is carried out at temperatures of from about 160 to about 200°C.

4. The process as claimed in at least one of claims 1 to 3, wherein the reaction in stage 1 is carried out in dimethylformamide, dimethylacetamide, dimethyl sulfoxide, tetramethylene sulfoxide, dimethyl sulfone, diphenyl sulfoxide, diphenyl sulfone, sulfolane, N-methylpyrrolidone or 1,3-dimethylimidazolin-2-one as the polar aprotic solvent.

5. The process as claimed in at least one of claims 1 to 4, wherein the reaction in stage 1 is carried out in the presence of a tetraalkyl(C₁-C₁₈)ammonium chloride or bromide, tetraalkyl(C₁-C₁₈)phosphonium chloride or bromide, tetraphenylphosphonium chloride or bromide or [(phenyl)ₘalkyl(C₁-C₁₈)ₙ]phosphonium chloride or bromide, where m is = 1 to 3, n is = 3 to 1 and m+n is = 4, as the phase transfer catalyst.

6. The process as claimed in at least one of claims 1 to 5, wherein the denitrating chlorination in stage 2 is carried out at temperatures of from about 100 to about 220°C.

7. The process as claimed in at least one of claims 1 to 6, wherein the denitrating chlorination in stage 2 is carried out continuously.

8. The process as claimed in at least one of claims 1 to 7, wherein the nitration in stage 3 is carried out using a mixed acid of 66% H₂SO₄ and 34% HNO₃ in oleum.

9. The process as claimed in at least one of claims 1 to 8, wherein the nitration in stage 3 is carried out at temperatures of from about 20 to about 50°C.

10. The process as claimed in at least one of claims 1 to 9, wherein glycols, polyhydric alcohols, monohydric aliphatic alcohols or formates are employed as reducing agents in stage 4.

11. The process as claimed in at least one of claims 1 to 10, wherein the reaction in stage 4 is carried out in a hydrogen atmosphere under pressure using hydrogen as reducing agent.

12. The process as claimed in at least one of claims 1 to 11, wherein the reaction is carried out in a hydrogen atmosphere under a pressure of from about 0.1 to about 50 bar using hydrogen as reducing agent.

13. The process as claimed in at least one of claims 1 to 12, wherein in stage 4 the palladium is employed on a support material.

14. The process as claimed in at least one of claims 1 to 13, wherein in stage 4 the palladium is employed on calcium carbonate, barium sulfate or active charcoal as support material.

15. The process as claimed in at least one of claims 1 to 14, wherein in stage 4 the palladium is used in quantities of from about 0.001 to about 50 mmol per mole of chlorine to be eliminated.

16. The process as claimed in at least one of claims 1 to 15, wherein in stage 4 the palladium is employed in concentrations of from about 0.5 to about 30% by weight, based on the support material used.

17. The process as claimed in at least one of claims 1 to 16, wherein the reduction in stage 4 is carried out in the presence of ammonia or of an hydroxide, oxide, carbonate or hydrogen carbonate of an alkali metal or of an alkaline earth metal, or mixtures thereof.

18. The process as claimed in at least one of claims 1 to 17, wherein the reduction in stage 4 is terminated after 90 to 95% conversion, and the crude solution thus obtained is fractionally distilled.

## Revendications

1. Procédé de préparation de la 3,5-difluoroaniline, caractérisé en ce que
(1) on fait réagir le 2,4,5-dichloronitrobenzène avec un fluorure de métal alcalin en présence ou en absence d'un solvant aprotique polaire à des températures comprises entre environ 100°C et environ 250°C et après filtration des sels précipités et distillation fractionnée de la solution brute,
(2) on transforme par réaction de chloration dénitrante le 5-chloro-2,4-difluoronitrobenzène obtenu en absence d'un acide de Lewis ou d'un autre catalyseur de chloration avec du chlore gazeux anhydre à des températures comprises entre environ 80 et environ 250°C pour donner le 1,3-dichloro-4,6-difluorobenzène,
(3) on transforme ce composé par une réaction de nitration dans de l'oléum avec un acide mixte (acide sulfurique/acide nitrique) à des températures comprises entre environ 15 et environ 80°C pour donner le 2,6-dichloro-3,5-difluoronitrobenzène et
(4) on réduit ce composé avec de l'hydrogène en présence de palladium en tant que catalyseur et en présence d'une base inorganique ou organique à des températures comprises entre environ 40 et environ 250°C.

2. Procédé selon la revendication 1, caractérisé en ce qu'on réalise l'étape 1 avec du fluorure de lithium, de sodium, de potassium, de rubidium ou de césium ou avec leurs mélanges.

3. procédé selon l'une au moins des revendications 1 et 2, caractérisé en ce qu'on réalise l'étape 1 à des températures comprises entre environ 160 et environ 200°C.

4. Procédé selon l'une au moins des revendications 1 à 3, caractérisé en ce qu'on réalise l'étape 1 dans du diméthylformamide, du diméthylacétamide, du diméthylsulfoxyde, du tétraméthylènesulfoxyde, de la diméthylsulfone, du diphénylsulfoxyde, de la diphénylsulfone, du sulfolane, de la N-méthylpyrrolidone ou de la 1,3-diméthylimidazoline-2-one en tant que solvant aprotique polaire.

5. Procédé selon l'une au moins des revendications 1 à 4, caractérisé à ce qu'on réalise l'étape 1 en présence d'un chlorure ou d'un bromure de (tétraalkyl en C₁-C₁₈)-ammonium, d'un chlorure ou d'un bromure de (tétra- alkyl en C₁-C₁₈)-phosphonium, du chlorure ou du bromure de tétraphénylphosphonium, d'un chlorure ou d'un bromure de [(phényl)ₘ(alkyl en C₁-C₁₈)ₙ]-phosphonium, m valant de 1 à 3, n valant de 3 à 1 et m+n valant 4, en tant que catalyseur de transfert de phase.

6. Procédé selon l'une au moins des revendications 1 à 5, caractérisé en ce qu'on réalise la réaction de chloration dénitrante de l'étape 2 à des températures comprises entre environ 100 et environ 220°C.

7. Procédé selon l'une au moins des revendications 1 à 6, caractérisé en ce qu'on réalise la réaction de chloration dénitrante de l'étape 2 en continu.

8. Procédé selon l'une au moins des revendications 1 à 7, caractérisé en ce qu'on réalise la réaction de nitration de l'étape 3 avec un acide mixte de 66% de H₂SO₄ et de 34 % de HNO₃ dans l'oléum.

9. Procédé selon l'une au moins des revendications 1 à 8, caractérisé en ce qu'on réalise la réaction de nitration de l'étape 3 à des températures comprises entre envion 20 et environ 50°C.

10. Procédé selon l'une au moins des revendications 1 à 9, caractérisé en ce qu'on utilise dans l'étape 4 des glycols, des poly-alcools, des mono-alcools aliphatiques ou des formiates en tant qu'agent de réduction.

11. Procédé selon l'une au moins des revendications 1 à 10, caractérisé en ce qu'on réalise l'étape 4 en utilisant de l'hydrogène en tant qu'agent de réduction sous une atmosphère d'hydrogène sous pression.

12. Procédé selon l'une au moins des revendications 1 à 11, caractérisé en ce qu'on travaille en utilisant de l'hydrogène en tant qu'agent de réduction sous une atmosphère d'hydrogène sous une pression comprise entre environ 0,1 et environ 50 bars.

13. Procédé selon l'une au moins des revendications 1 à 12, caractérisé en ce qu'on utilise dans l'étape 4 du palladium sur un matériau support.

14. Procédé selon l'une au moins des revendications 1 à 13, caractérisé en ce qu'on utilise dans l'étape 4 du palladium sur du carbonate de calcium, du sulfate de barium ou sur du charbon actif en tant que matériau support.

15. Procédé selon l'une au moins des revendications 1 à 14, caractérisé en ce qu'on utilise dans l'étape 4 du palladium sous une quantité d'environ 0,001 à environ 50 mmol. par mole de chlore à éliminer.

16. Procédé selon l'une au moins des revendications 1 à 15, caractérisé en ce qu'on utilise dans l'étape 4 du palladium dans une concentration d'environ 0,5 à environ 30% en poids, rapporté au matériau support appliqué.

17. Procédé selon l'une au moins des revendications 1 à 16, caractérisé en ce qu'on réalise la réaction de réduction de l'étape 4 en présence d'ammoniaque (ou ammoniac) ou un hydroxyde, un oxyde, un carbonate ou un hydrogénocarbonate d'un métal alcalin ou d'un métal alcalino-terreux ou de leurs mélanges.

18. Procédé selon l'une au moins des revendications 1 à 17, caractérisé en ce qu'on arrête la réduction de l'étape 4 après un état d'avancement de 90 à 95% et on distille par fractionnement la solution brute ainsi obtenue.
